# EUROPEAN PATENT APPLICATION

(11) **EP 4 400 580 A1**
(43) Date of publication of application: **17.07.2024**
(21) Application number: 22867310.9
(22) Date of filing: 02.09.2022
(51) Int. Cl.: C12N 5/077, C12N 1/04, C12Q 1/02, G01N 33/15, A61L 27/38, A61K 35/22

(54) **STORAGE METHOD AND TRANSPORTATION METHOD OF RENAL CELLS, RENAL CELLS, DRUG EVALUATION SYSTEM AND CELL PRODUCT**

(30) Priority: 10.09.2021 JP 2021148155
(71) Applicant: Nikkiso Co., Ltd., Tokyo 150-6022 (JP)
(72) Inventor: TAKAHASHI, Etsushi, Kanazawa-shi, Ishikawa 920-0177 (JP)
(74) Representative: Hoffmann Eitle
(86) International application number: PCT/JP2022/033176
(87) International publication number: WO 2023/037988

(57) **Abstract**

Provided is a method for storing or transporting renal cells that have a high cell viability, maintain a physiological function of the kidney, and can be used as a drug evaluation system, at a temperature lower than a culture temperature. An aspect of the present invention is a method for storing or transporting renal cells, the method including a maintenance step of managing a liquid containing aggregates of the renal cells to a temperature that is a maintenance temperature and lower than a culture temperature of the renal cells and at which the liquid is not frozen.

## Description

### Technical Field

The present invention relates to a method for storing renal cells and a method for transporting renal cells. The present invention further relates to a cell product containing renal cells.

### Background Art

A drug administered to a living body is absorbed into the living body, and then excreted from the blood into the urine through the proximal tubule in the kidney. Therefore, renal damage is often caused by the nephrotoxicity of the drug. It is very important in drug discovery research to examine pharmacokinetics in the kidney in order to determine the action of the drug. For this reason, development of a drug discovery support device capable of evaluating pharmacokinetics and toxicity using renal cells is desired. In addition, such a drug discovery support device is also useful for the development of a therapeutic agent for a disease related to the kidney (for example, kidney cancer, hyperuricemia, and the like).

When the drug discovery support device using such cells is supplied or used, a method for stably storing or transporting the cells is required. Generally, cells are twodimensionally planar cultured in flat bottom culture plates. Conventionally, there has been a problem that in order to maintain a normal cell state, a temperature management device for maintaining a vessel containing cells at a culture temperature is required, resulting in high the cost of storage or transportation of cells.

### Citation List

### Patent Literature

Patent Literature 1: JP 2015-50992 A

### Summary of Invention

### Technical Problem

The present inventor has obtained a finding that when planar cultured renal cells are maintained at a temperature lower than the culture temperature for a certain period of time, the morphology of the cells is changed, and the cell viability is reduced. Therefore, based on the findings, an object of the present invention is to provide a method for storing or transporting renal cells having a high cell viability and maintaining the physiological functions of the cells at a temperature lower than the culture temperature.

### Solution to Problem

As a result of various studies to solve the above problems, it has been found that by forming one or more aggregates of renal cells, it is possible to maintain the physiological functions of the cells at a temperature lower than the culture temperature for a certain period of time.

One embodiment according to the present invention is a method for storing or transporting renal cells, including a maintenance step of managing a liquid containing one or more aggregates of renal cells to a temperature that is lower than a culture temperature of the cells and at which the liquid is not frozen.

One embodiment according to the present invention is renal cells stored or transported by a method including a maintenance step of managing a liquid containing one or more aggregates of renal cells to a temperature that is lower than the culture temperature of the cells and at which the liquid is not frozen.

### Advantageous Effects of Invention

According to the present invention, there is provided a method for storing or transporting renal cells having a high cell viability and maintaining a physiological function of the kidney at a temperature that is lower than a culture temperature.

### Brief Description of Drawings

Fig. 1 is a block diagram showing a schematic configuration of a renal cell storage and transportation system.
Fig. 2 is a flowchart showing an example of an operation of the renal cell storage and transportation system.
Fig. 3-1(a) to Fig. 3-1(c) are optical microscopic images of a proximal tubule epithelial cell aggregate under 37°C conditions, room temperature conditions, and refrigeration conditions, respectively.
Fig. 3-2 is an ATP amount (cell viability) of a proximal tubule epithelial cell aggregate after 24 hours of standing.
Fig. 3-3(a) to Fig. 3-3(d) are ATP amounts (cell viability) of a proximal tubule epithelial cell aggregate after 48 hours of standing, 72 hours of standing, 144 hours of standing, and 240 hours of standing, respectively.
Fig. 4-1 shows the results of OAT1 gene expression analysis by a real-time PCR method of a proximal tubule epithelial cell aggregate after 72 hours of room temperature standing, in comparison with human renal cortex.
Fig. 4-2 shows the results of OCT2 gene expression analysis by a real-time PCR method of a proximal tubule epithelial cell aggregate after 72 hours of room temperature standing, in comparison with human renal cortex.
Fig. 4-3 shows the results of URAT1 gene expression analysis by a real-time PCR method of a proximal tubule epithelial cell aggregate after 72 hours of room temperature standing, in comparison with human renal cortex.
Fig. 4-4 shows the results of OAT1 gene expression analysis by a real-time PCR method of a proximal tubule epithelial cell aggregate after 24 hours of refrigeration standing, in comparison with human renal cortex.

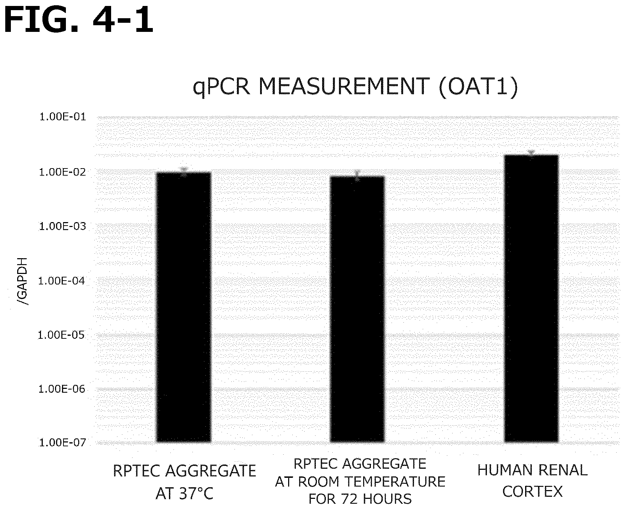

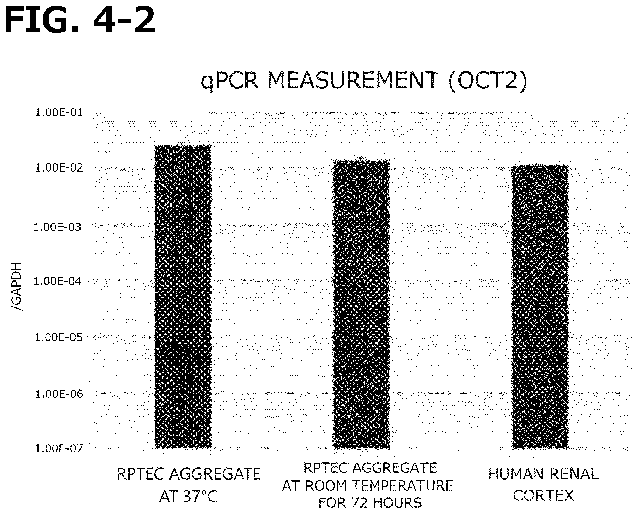

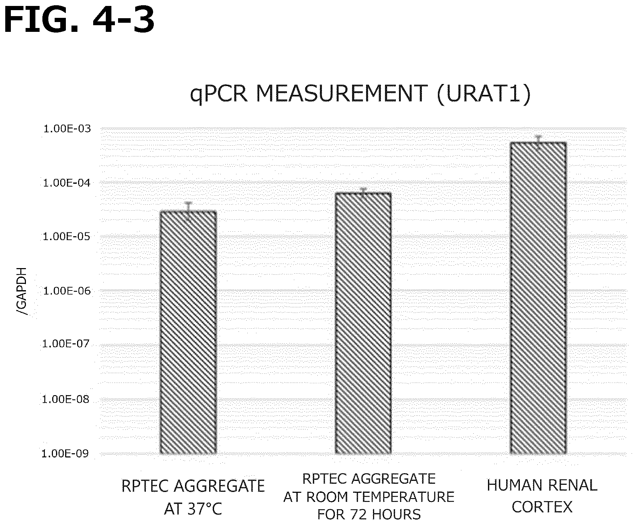

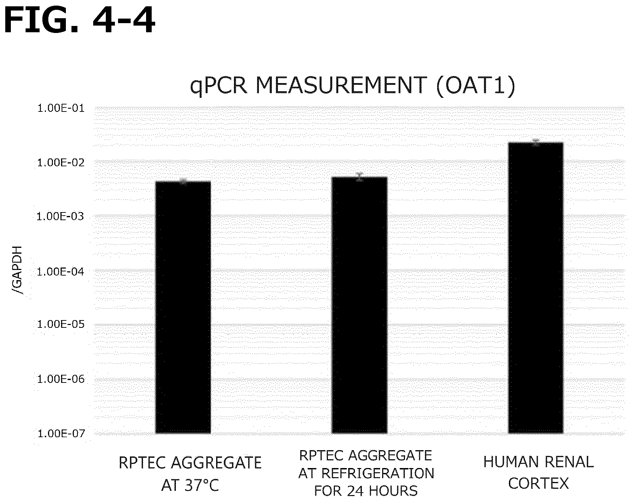

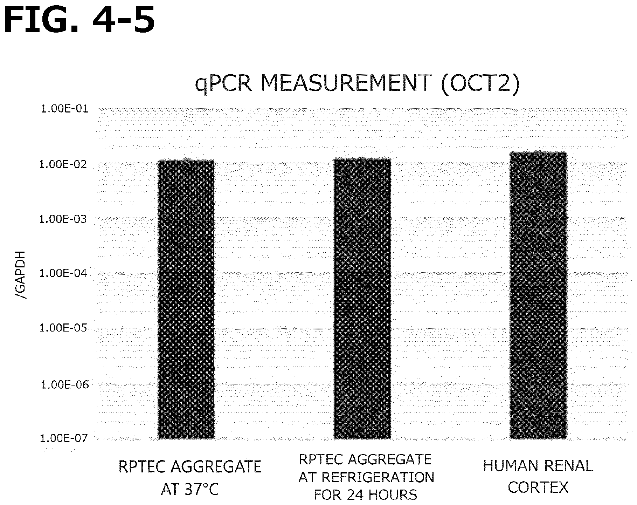

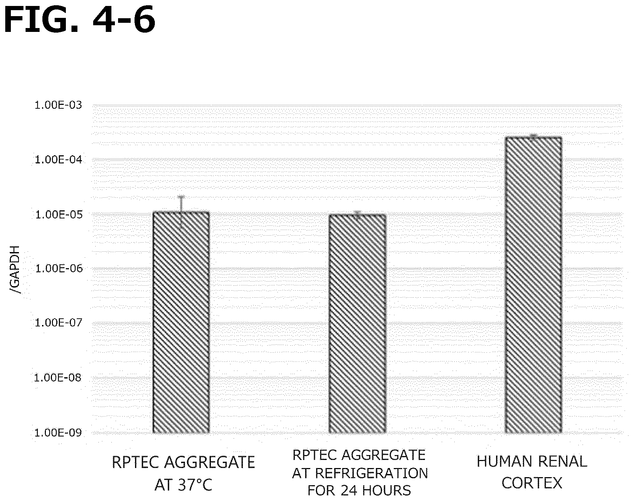

Fig. 4-5 shows the results of OCT2 gene expression analysis by a real-time PCR method of a proximal tubule epithelial cell aggregate after 24 hours of refrigeration standing, in comparison with human renal cortex.
Fig. 4-6 shows the results of URAT1 gene expression analysis by a real-time PCR method of a proximal tubule epithelial cell aggregate after 24 hours of refrigeration standing, in comparison with human renal cortex.

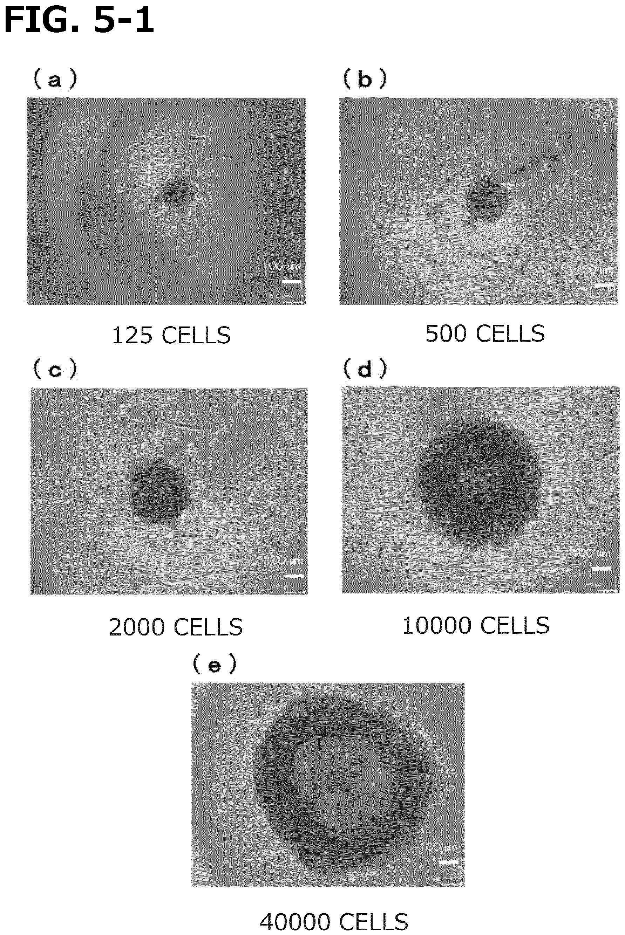

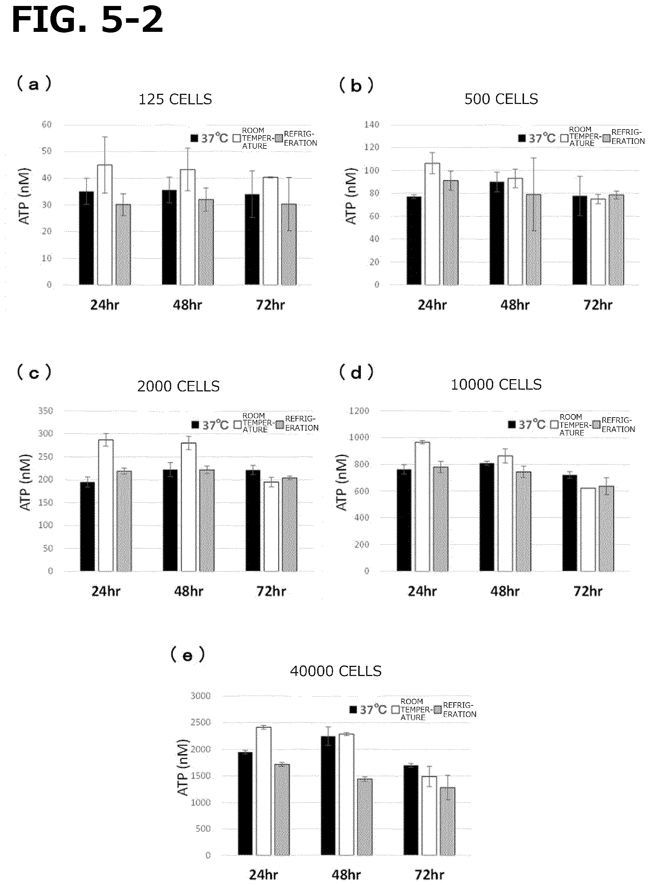

Fig. 5-1(a) to Fig. 5-1(e) are optical microscopic images of a proximal tubule epithelial cell aggregate in the cell number of 125 cells, 500 cells, 2000 cells, 10,000 cells, and 40,000 cells, respectively.
Fig. 5-2(a) to Fig. 5-2(e) are ATP amounts (cell viability) of a proximal tubule epithelial cell aggregate in the cell number of 125 cells, 500 cells, 2000 cells, 10,000 cells, and 40,000 cells, respectively.

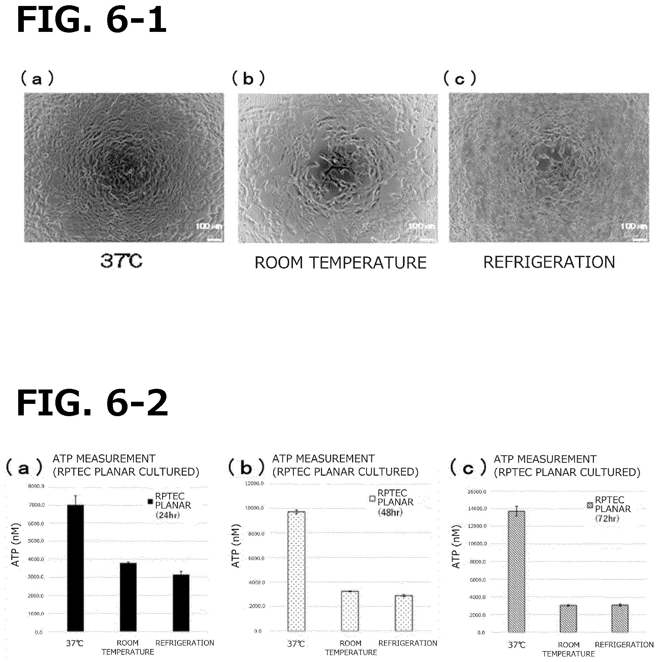

Fig. 6-1(a) to Fig. 6-1(c) are optical microscopic images of planar cultured proximal tubule epithelial cells under 37°C conditions, room temperature conditions, and refrigeration conditions, respectively.
Fig. 6-2(a) to Fig. 6-2(c) are ATP amounts (cell viability) of the planar cultured proximal tubule epithelial cells at elapsed times of 24 hours, 48 hours, and 72 hours, respectively.

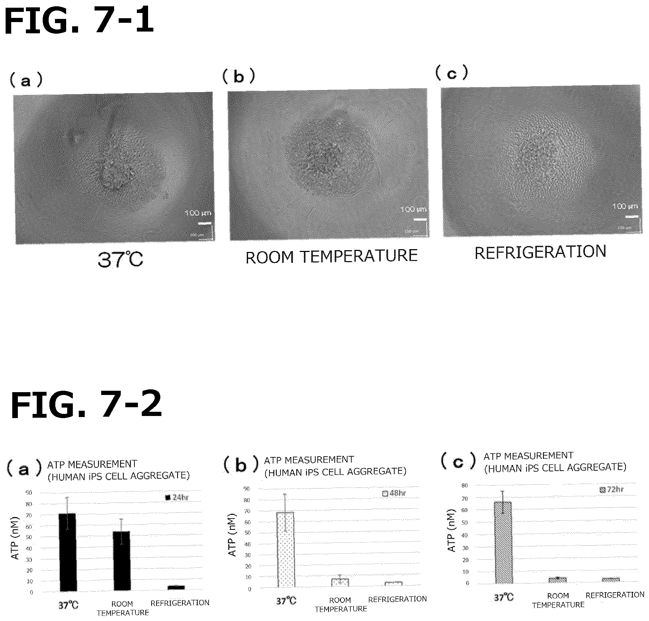

Fig. 7-1(a) to Fig. 7-1(c) are optical microscopic images of a human iPS cell aggregate under 37°C conditions, room temperature conditions, and refrigeration conditions, respectively.
Fig. 7-2(a) to Fig. 7-2(c) are ATP amounts (cell viability) of a human iPS cell aggregate at elapsed times of 24 hours, 48 hours, and 72 hours, respectively.

### Description of Embodiments

Hereinafter, the present invention will be described in detail.

The method for storing or transporting renal cells according to one embodiment of the present invention includes a maintenance step of managing a liquid containing one or more aggregates of the renal cells to a temperature that is lower than a culture temperature of the cells and at which the liquid is not frozen.

The renal cells used in the present invention may be cultured, and the source thereof is not limited. The renal cells are preferably derived from mammals, and are preferably derived from primates such as humans and monkeys. Depending on the purpose, it may be derived from normal kidney or kidney having a disease. Examples of the renal cells include cells constituting epithelium, cortex, proximal tubule, distal tubule, collecting duct, glomerulus, and the like, specifically, proximal tubule epithelial cells (RPTEC), mesangial cells, and the like. The renal cells may be primary cells or renal cells derived from stem cells such as iPS cells or ES cells. In addition, the renal cells may be immortalized renal cells, established cells (HK-2 cells and the like), cells derived from other animal species (MDCK cells, LLC-PK1 cells, JTC-12 cells, and the like), and forcibly expression cells obtained by introducing a gene into the renal cells in order to express a protein such as a specific transporter. More specifically, examples of the renal cells include human proximal tubule epithelial cells, human distal tubule epithelial cells, and human collecting duct epithelial cells collected and isolated from the kidney, and proximal tubule epithelial cells, distal tubule epithelial cells, and collecting duct epithelial cells induced to differentiate from human iPS cells or human ES cells. For use in drug discovery research, proximal tubule epithelial cells are preferred, particularly proximal tubule epithelial cells derived from human normal kidney.

By using a culture medium and a culture vessel suitable for cells to be cultured, the renal cells can be cultured according to a conventional method, for example, under conditions of 37°C and 5% CO₂. The culture may be any of static culture, shaking culture, stirring culture, and the like. The culture may be adhesion culture, but it is preferable to perform culture in a non-adhesion state (for example, suspension culture) for at least a part of the period. The renal cells can be cultured in a non-adhesion state to a culture vessel to form an aggregate. The "non-adhesion state" refers to a state in which all or most of the cells are not adhered to the surface of the culture vessel, and includes a state in which all or most of the cells are separated from the surface of the culture vessel and a state in which when the cells are in contact with the surface of the culture vessel, the cells can still be easily separated from the surface of the culture vessel by coating of the culture vessel, convection of the culture medium, or the like without using a tool, an enzyme, or the like.

For example, in some cases, an aggregate of renal cells are formed within 24 hours after initiation of culture of the renal cells. Then, by culturing renal cells in an aggregate state for a part of the period, it is possible to restore the physiological function of renal cells that have been dedifferentiated and lowered. It is generally desirable to culture renal cells in a non-adhesion state in a culture vessel for a 120-hour period or longer. This makes it possible to obtain cultured renal cells in a state of higher expression of physiological functions. During the culture period, the culture medium is preferably replaced periodically. For example, the culture medium is replaced every two days.

As the culture medium, any known culture medium can be appropriately used. For example, in a case of culture of proximal tubule epithelial cells, a commercially available tubular cell culture medium can be used, and examples of preferred culture mediums include REGM (registered trademark) (Lonza Group AG), EpiCM (registered trademark) (ScienCell Research Laboratories, Inc.), and Keratinocyte SFM (registered trademark) (Thermo Fisher Scientific Inc.).

In addition, conventionally known materials and additives useful for cell culture can be appropriately used. For example, collagen I (type I collagen) can be added to the culture medium. Collagen I has an action of adhering renal cells to each other. Therefore, the formation of an aggregate is promoted by culturing renal cells in a culture medium containing collagen I. Collagen I is preferably full-length collagen I, and may be an α1 chain or an α2 chain constituting collagen I, and further a collagen peptide obtained by fragmenting each chain, or the like. The source of collagen I is not particularly limited, and collagen I may be derived from human or other animals.

Any culture vessel can be used, and in order to promote the formation of the aggregate, it is preferable that the culture vessel is subjected to a cell non-(low) adhesion treatment or is made of a cell non-(low) adhesion material. Examples of the cell non-(low) adhesion treatment include a cell non-adhesion hydrogel coating treatment to the surface of the vessel, a 2-methacryloyloxyethyl phosphorylcholine (MPC) coating treatment, a proteosave (registered trademark) SS coating treatment, and a mirror polishing treatment. Examples of the cell non-(low) adhesion material include glass, and polymeric materials such as low-density polyethylene, mediumdensity polyethylene, polyvinyl chloride, polyethylene-vinyl acetate copolymer, poly (ethylene-ethyl acrylate) copolymer, poly (ethylene-methacrylate) copolymer, poly (ethylene-vinyl acetate) copolymer, and mixtures of two or more of these polymers.

In a case where a large amount of aggregates are formed, a high-density spheroid production plate or dish can be used. Furthermore, a culture vessel such as a spinner flask may be used as necessary. For example, it is preferable to use a culture vessel of ELPLASIA (registered trademark) series (Corning Incorporated), a culture vessel of EZSPHERE (registered trademark) series (AGC Techno Glass Co., Ltd.), or the like. There are types of these culture vessels such as 6 well plates, 24 well plates, 96 well plates, 384 well plates, dishes of various sizes, and the number of aggregates that can be produced varies depending on the size of the bottom area of the vessel. For example, when a 96 well plate (V bottom), a 96 well plate (U bottom), or a 384 well plate (U bottom) subjected to low adhesion treatment is used, one aggregate is formed in one well.

Aggregates produced using high-density spheroid production plates or dishes or the like can be collected and cultured with suspension shaking. In a case of being cultured with suspension shaking, it is preferable to culture an aggregate by placing a dish, a plate, or the like subjected to the cell non-(low) adhesion treatment on a shaker. As the shaker, a reciprocating shaker and a swirling shaker can be used.

As used herein, the "aggregate" of cells refers to a blocky collection of several or more cells. It is also referred to as agglomerates and spheroids. The cell number constituting an aggregate is, for example, 5 cells or more, 25 cells or more, 50 cells or more, preferably 100 cells or more, more preferably 125 cells or more, and still more preferably 500 cells or more. The cell number constituting an aggregate is, for example, 40,000 cells or less, preferably 10,000 cells or less, more preferably 2000 cells or less, or 1000 cells or less. When the cell number constituting an aggregate is in such a range, there is little variation in aggregate production, and aggregates are difficult to join with each other, and therefore the aggregate size tends to be uniform. In addition, cells tend to maintain high viability.

The size of the aggregate can be controlled by adjusting the cell number seeded in the culture vessel. For example, when cultured in a multi-well plate, one aggregate is formed in one well. Therefore, when the number of renal cells seeded per well is 125 cells or more and 10,000 cells or less, the number of renal cells constituting the aggregate is 125 cells or more and 10,000 cells or less.

The diameter of the aggregate is preferably, for example, 100 µm or more and 800 µm or less. The volume of the aggregate is preferably, for example, 0.001 mm³ or more and 0.300 mm³ or less.

Note that the diameter of the aggregate is defined as the maximum width of the aggregate. That is, the diameter of the aggregate is the length of the largest one of the straight lines connecting two points on the outer edge of the aggregate. In addition, since the aggregate is substantially spherical, the volume of the aggregate can be calculated from the measured diameter. The diameter of the aggregate can be measured using, for example, a photograph taken with a phase contrast microscope. BZ-X 710 (Keyence Corporation) can be used as the phase contrast microscope, and analysis software can be used to measure the diameter.

The liquid used in storing or transporting the renal cells is not particularly limited as long as it does not adversely affect the cells, and may be a culture medium when an aggregate is produced, a fresh culture medium, or a liquid other than the culture medium (for example, physiological saline or a buffer solution). The liquid may contain salts, buffer agents, serum, vitamins, amino acids, glucose (sugar), electrolytes, antibiotics, growth factors (compounds, proteins). It is preferable that the liquid is replaced periodically in a case where storage or transportation lasts for 48 hours or longer. For example, the liquid is replaced every two days.

As the storage and transportation container of a liquid containing an aggregate of renal cells, any vessel can be used similarly to the culture vessel described above, but it is preferable that the vessel is subjected to a cell non-(low) adhesion treatment or is made of a cell non-(low) adhesion material.

A method for storing or transporting renal cells according to one embodiment of the present invention includes a maintenance step of managing a liquid containing an aggregate of renal cells to a temperature (hereinafter, the temperature may be referred to as a "maintenance temperature" for convenience) that is lower than a culture temperature of the renal cells and at which the liquid is not frozen when storing or transporting the renal cells.

As the temperature that is lower than the culture temperature of the renal cells and at which the liquid is not frozen, the optimum temperature can be appropriately selected according to the type and use of the cells and/or the period required for storage or transportation. Note that the culture temperature is a temperature suitable for the survival of cells, and the cells may or may not be grown. Since the culture temperature of the cells is typically 37°C, it is desirable that the maintenance temperature is lower than this. On the other hand, when the maintenance temperature is too low, partial freezing occurs in the cells, and thus the cell viability of an aggregate may be reduced. Therefore, it is desirable that the maintenance temperature is 0°C or more. The maintenance temperature can be, for example, 0°C or more and lower than 37°C, preferably 10°C or more and lower than 37°C, more preferably 20°C or more and lower than 28°C in a case where it is desirable to maintain the temperature at a relatively high temperature, and preferably 0°C or more and lower than 10°C, more preferably 3°C or more and lower than 8°C in a case where it is desirable to maintain the temperature at a relatively low temperature. Note that in the present description, for convenience, a temperature range of 10°C or more and lower than 37°C may be referred to as "room temperature", and a temperature range of 0°C or more and lower than 10°C may be referred to as "refrigeration".

In a case of storage, an incubator, a thermostatic chamber, a refrigerator, or the like that can be adjusted to a desired temperature can be used in the maintenance step.

In a case of transportation, it is preferable to use a heat-insulating container such as foam polystyrene in the maintenance step. The size of the heat-insulating container is not particularly limited as long as the storage and transportation container can be packaged. When there is a gap between the heat-insulating container and the storage and transportation container, a cushioning material is preferably packed so that the storage and transportation container does not move. As the heat-insulating container, a paper container or a corrugate cardboard container can also be used.

Note that the maintenance step is not necessarily performed by standing. Therefore, it can be performed on a shaker or under a situation where there is a vibration that does not cause breakdown of an aggregate, such as during transportation.

During storage or transportation of renal cells, the number of aggregates in the liquid can be one in order to prevent the aggregates from joining to be non-uniform in size. At this time, the lower limit value of the volume ratio of an aggregate to the liquid is preferably 0.001% or more, 0.002% or more, or 0.003% or more. The upper limit value of the volume ratio of an aggregate to the liquid is preferably 0.800% or less, 0.500% or less, or 0.200% or less. When the volume ratio of an aggregate to the liquid is in such a range, it is possible to prevent the aggregate from being exposed to the air due to drying of the liquid during storage or transportation, and oxygen is sufficiently supplied to the aggregate.

When the renal cells are stored or transported, the lower limit value of the specific gravity of an aggregate to the liquid is preferably 1.00 or more. With such a specific gravity, since the aggregate can exist in the liquid without floating on the liquid surface, the aggregate is less likely to be affected by a change in the extracellular environment. Therefore, the upper limit value of the specific gravity of an aggregate is preferably 1.20 or less, 1.15 or less, or 1.10 or less.

A maintenance period of the maintenance step is a period in which the cell viability of the aggregate of the renal cells is a predetermined value or more. The cell viability is a ratio of the number of viable cells at the maintenance temperature to the number of viable cells at the culture temperature of renal cells. The cell viability is preferably 80% or more, and more preferably 90% or more. Renal cells having such cell viability can be utilized for drug evaluation.

The present inventor has found that there is the following relationship between the maintenance period and the maintenance temperature in the maintenance step.

That is, the maintainable period during which the cell viability can be maintained at a predetermined value or more is calculated based on the following formula. (Maintainable period (days)) = A/{(culture temperature (°C)) - (maintenance temperature (°C))}

Here, A: a coefficient determined according to the predetermined value and the cell number of the aggregate (day ·°C)

From the above formula, the upper limit value of the maintenance period according to the desired maintenance temperature and cell viability can be calculated.

Similarly, the maintainable temperature at which the cell viability can be maintained at a predetermined value or more is calculated based on the following formula. (Maintainable period (days)) = A/{(culture temperature (°C)) - (maintainable temperature (°C))}

Here, A: a coefficient determined according to the predetermined value and the cell number of the aggregate (day ·°C)

From the above formula, the lower limit value of the maintenance temperature according to the desired maintenance period and cell viability can be calculated.

Here, for example, when the predetermined value is 90% and the cell number of the aggregate is 1000 cells, the value of A can be set to 34.5, and when the predetermined value is 80% and the cell number of the aggregate is 1000 cells, the value of A can be set to 69.

From the above, for example, when the cell number of the aggregate is 1000 cells, in a case where the culture temperature is 37°C and the maintenance temperature is 25°C, the maintainable period is 5.75 days when the predetermined value is 80%, and 2.88 days when the predetermined value is 90%. That is, in the case where the culture temperature is 37°C and the maintenance temperature is 25°C, the maintenance period is preferably 5.75 days or less, and more preferably 2.88 days or less.

The renal cell according to one embodiment of the present invention has a higher physiological function of the kidney or is functionally equivalent compared to the human renal cortex.

Being "functionally equivalent compared to the human renal cortex" is meant exhibiting at least a level of expression equivalent to the human renal cortex, with respect to one or more gene expression associated with a physiological function of the kidney expressed in the human renal cortex.

Examples of genes related to the physiological function of the kidney include AQP1, CD13, SGLT2, Na/K ATPase, URAT1, PEPT1, MDR1, OAT1, OCT2, OCTN2, E-cadherin, and ZO-1. AQP1 (aquaporin1) is a gene encoding a protein involved in water transport. CD13 (alanyl aminopeptidase) is a gene encoding a protein involved in peptidization of a protein. SGLT2 (sodium glucose cotransporter2) is a gene encoding a protein involved in transport of sodium and glucose. Na/K ATPase is a gene encoding a protein involved in ion transport. URAT1 (urate transporter 1) is a gene encoding a protein involved in uric acid resorption. PEPT1 (peptide transporter 1) is a gene encoding a protein involved in peptide transport transportation. MDR1 (multiple drug resistance 1), OAT1 (organic anion transporter 1), OCT2 (organic cation transporter 2), and OCTN2 (organic cation transporter novel 2) are genes encoding proteins involved in drug transportation. E-cadherin and ZO-1 (zonula occludens-1) are genes encoding proteins involved in intercellular binding.

The expression level of one or more of these genes in the human renal cortex and the kidney cell culture can be measured by a general real-time PCR method (qPCR method), and whether or not both are equivalent can be determined by comparing them. Note that when used for determination, measured values of two or more experiments are averaged and used.

For example, in the renal cells stored or transported for a predetermined period, in a case where the expression level of any of the above genes is 10% or more of the expression level of the gene in the human renal cortex, the renal cells are determined to be functionally equivalent to the human renal cortex. In the kidney cell culture functionally equivalent to the human renal cortex, the expression level of one of the above genes is preferably 10% or more, more preferably 25% or more of the expression level in the human renal cortex. Alternatively, in renal cells functionally equivalent to the human renal cortex, the expression levels of the two or more genes are preferably 10% or more, more preferably 25% or more of the expression levels in the human renal cortex.

The renal cells according to one embodiment of the present invention can be provided as a drug evaluation system or a cell product. Examples of the drug evaluation system include a system for evaluating pharmacokinetics and nephrotoxicity in renal cells. In addition, such renal cells can also be used as a mechanism analysis and a therapeutic agent search tool of diseases related to the kidney such as kidney cancer and hyperuricemia.

### (Renal cell storage and transportation system)

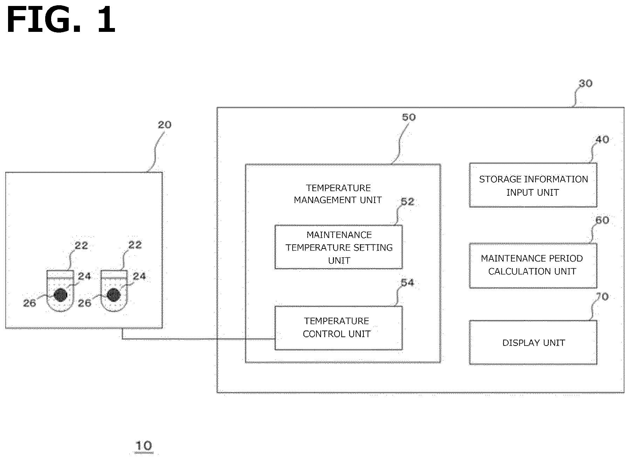

Fig. 1 is a block diagram showing a schematic configuration of a renal cell storage system 10 according to an embodiment.

As shown in Fig. 1, the renal cell storage system 10 includes a renal cell storage device 20 and a temperature management device 30.

The renal cell storage device 20 is a storage chamber capable of maintaining the temperature in the chamber at a predetermined temperature, and is, for example, an incubator, a thermostatic chamber, a refrigerator, or the like. A culture vessel 22 is stored in the chamber of the renal cell storage device 20. The culture vessel 22 contains a liquid 24 and an aggregate 26 of renal cells. Note that when the temperature in the renal cell storage device 20 is in a constant temperature state at a predetermined maintenance temperature, the temperature of the liquid 24 in the culture vessel 22 can be regarded as the predetermined maintenance temperature.

The temperature management device 30 includes a storage information input unit 40, a temperature management unit 50, a maintenance period calculation unit 60, and a display unit 70. The temperature management device 30 can be built in the renal cell storage device 20.

Information on the liquid 24 in the culture vessel 22, information on the culture temperature of renal cells, information on the maintenance temperature of the liquid 24, information on the storage period (maintenance period) of renal cells, information on the cell viability, information on the cell number of the aggregate, and the like can be input to the storage information input unit 40 by the user.

Furthermore, information on the component and concentration (or content) of the liquid 24 in which an aggregate 26 is stored may be input to the storage information input unit 40. For example, in a case where the liquid is physiological saline, "sodium chloride" is input as a component, and further, the concentration of "sodium chloride" is input. Furthermore, a temperature at which the liquid 24 is frozen may be input to the storage information input unit 40.

The temperature management unit 50 includes a maintenance temperature setting unit 52 and a temperature control unit 54.

The maintenance temperature setting unit 52 sets the maintenance temperature in the renal cell storage device 20, that is, sets the maintenance temperature of the liquid 24 in the culture vessel 22 to a temperature lower than the culture temperature of the renal cells input to the storage information input unit 40 and at which the liquid 24 is not frozen. The temperature at which the liquid 24 is not frozen may be a temperature higher than the temperature at which the liquid 24 input to the storage information input unit 40 is frozen, or may be calculated from the component and concentration of the liquid 24 in consideration of the freezing point depression.

Specifically, the maintenance temperature may be set by the user via the storage information input unit 40, or may be determined according to a desired cell viability, the desired cell number of the aggregate, a desired maintenance period, and the like.

In a case where the maintenance temperature is set according to the cell viability, the cell number of the aggregate, and the like, the maintenance temperature is set to be equal to or higher than the maintainable temperature calculated based on the following formula. In other words, the lower limit value of the maintenance temperature is determined according to the maintenance period that is a period during which the renal cells are stored. (Maintainable period (days)) = A/{(culture temperature (°C)) - (maintainable temperature (°C))}

Here, A: a coefficient determined according to the predetermined value and the cell number of the aggregate (day ·°C)

Here, the maintainable temperature is a temperature at which the cell viability, which is the ratio of the number of viable cells at the maintenance temperature to the number of viable cells at the culture temperature of renal cells, can be maintained at a predetermined value or more.

The temperature control unit 54 controls the temperature in the renal cell storage device 20 so as to be the maintenance temperature set by the maintenance temperature setting unit 52. Specifically, the temperature control unit 54 and the temperature management device 30 are connected to communicate with each other in a wireless or wired manner, and the temperature control unit 54 transmits information on the maintenance temperature to the temperature management device 30. The renal cell storage device 20 that has received the information on the maintenance temperature performs temperature management so that the temperature in the chamber becomes the maintenance temperature.

Note that the temperature control in the renal cell storage device 20 may be started after the culture vessel 22 is stored in the renal cell storage device 20, and the temperature control in the renal cell storage device 20 may be started before the culture vessel 22 is stored in the renal cell storage device 20. After the temperature control in the renal cell storage device 20 is performed in advance, the culture vessel 22 is stored in the renal cell storage device 20, so that the temperature of the liquid 24 in the culture vessel 22 can be quickly brought to the desired maintenance temperature.

The maintenance period calculation unit 60 sets a maintenance period for maintaining the temperature of the liquid at the maintenance temperature by the renal cell storage device 20 to be equal to or less than a maintainable period (days) calculated based on the following formula, as necessary. In other words, the upper limit value of the maintenance period is determined according to the maintenance temperature. (Maintainable period (days)) = A/{(culture temperature (°C)) - (maintenance temperature (°C))}

Here, A: a coefficient determined according to the predetermined value and the cell number of the aggregate (day ·°C)

Here, the maintainable period (days) is a period in which the cell viability, which is the ratio of the number of viable cells at the maintenance temperature to the number of viable cells at the culture temperature of renal cells, can be maintained at a predetermined value or more.

The display unit 70 displays necessary information on a display (not illustrated). Examples of the information displayed by the display unit 70 include the maintenance period (maintenance (storage) start date and time and maintenance (storage) end date and time) calculated by the maintenance period calculation unit 60, the maintenance temperature set by the maintenance temperature setting unit 52, and the like.

The renal cell storage system 10 can be installed in a transportation means such as a truck, a cargo vehicle, or a ship. In this case, the renal cell storage system 10 can be reread as the renal cell transportation system 10.

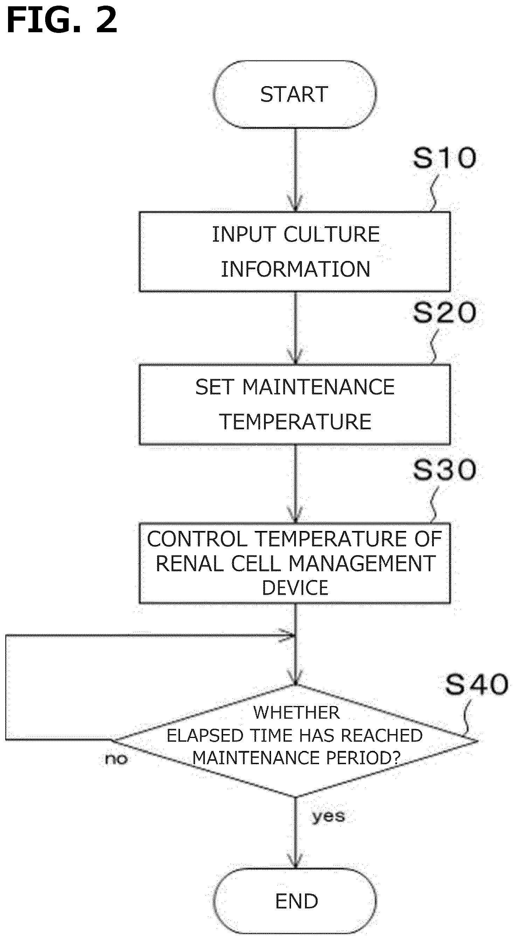

Fig. 2 is a flowchart showing an example of the operation of the renal cell storage system 10. The following example is a case where the maintenance temperature is set according to the maintenance period of the renal cells.

First, the user inputs information on storage of renal cells to the storage information input unit 40. Specifically, the maintenance period of the renal cells, the culture temperature of the renal cells, and the temperature at which the liquid is frozen are input (S10).

Next, the maintenance temperature setting unit 52 sets the maintenance temperature of the liquid 24 according to the setting method described above (S20). Note that it is set as a condition that the maintenance temperature is lower than the culture temperature of renal cells and equal to or lower than the freezing temperature of the liquid 24.

Next, in a state where the culture vessel 22 is stored in the renal cell storage device 20, the temperature control unit 54 controls the temperature in the renal cell storage device 20, that is, the temperature of the liquid 24 to be the maintenance temperature (S30).

Next, it is determined whether or not the time elapsed from the time point when the temperature in the renal cell storage device 20 is set to the maintenance temperature has reached the maintenance period (S40). In a case where the elapsed time has reached the maintenance period (S40, yes), the storage or temperature management of the renal cells is terminated. In a case where the elapsed time has not reached the maintenance period (S40, no), the process returns to the determination in S40.

One aspect of the present invention is a renal cell storage or transportation system.

The renal cell storage or transportation system includes:
a cell storage device that stores a culture vessel containing a liquid and an aggregate of renal cells and is capable of maintaining a temperature of the liquid at a predetermined temperature; and
a temperature management device that maintains a temperature of a liquid maintained by the cell storage device so as to be a maintenance temperature that is lower than a culture temperature of renal cells and at which the liquid is not frozen. In the above aspect, a lower limit value of the maintenance temperature may be determined according to a maintenance period that is a period during which the renal cells are stored.

In addition, a renal cell storage or transportation system according to another aspect of the present invention includes:
a cell storage device that stores a culture vessel containing a liquid and an aggregate of renal cells and is capable of maintaining a temperature of the liquid at a predetermined temperature; and
a temperature management device that maintains a temperature of a liquid maintained by the cell storage device so as to be a maintenance temperature that is lower than a culture temperature of renal cells and at which the liquid is not frozen, in which
an upper limit value of a maintenance period may be determined according to the maintenance temperature.

According to one embodiment of the present invention, the upper limit value of the maintenance period can be calculated according to the maintenance temperature of the renal cells, or the lower limit value of the maintenance temperature can be calculated according to the maintenance period. That is, one embodiment according to the present invention can be used for management of a maintenance temperature and a maintenance period as a renal cell storage and transportation system.

The present invention is not limited to the above-described embodiments, and various modifications such as design changes can be made on the basis of knowledge of those skilled in the art, and embodiments to which such modifications are made are also included in the scope of the present invention.

### Examples

### 1. Effects of temperature on proximal tubule epithelial cell aggregate

### <1-1. Morphology and cell viability of aggregate>

Human proximal tubule epithelial cells {Clonetics (registered trademark), catalog number CC-2553, RPTEC-renal proximal tubule epithelial cells} obtained from Lonza Group AG were used as renal cells. The frozen vials stored in a liquid nitrogen storage were thawed by immersing them in a thermostatic bath at 37°C. After thawing, the cell suspension in the frozen vial was admixed with the recommended culture medium {REGM (registered trademark), Lonza Group AG} and cultured in a culture dish. The cells were cultured under conditions of 37°C and 5% CO₂ while replacing the culture medium at a frequency of once every two days. The cells were collected before becoming confluent, and seeded on a 96 well V-bottom plate {PrimeSurface (registered trademark) plate 96 V, Sumitomo Bakelite Co., Ltd.} subjected to cell low adhesion treatment so that the cell number was 1000 cells per well, and cultured to form an aggregate. The aggregate was cultured while replacing the culture medium at a frequency of once every two days. The term "confluent" means that the ratio of the area occupied by cells to the entire culture surface of the culture vessel is about 100%, that is, means a state in which cells have grown to the full extent of the culture surface without gaps.

After culturing for 240 hours or longer, the plate containing the culture solution containing the aggregate was allowed to stand under the room temperature conditions or under the refrigeration conditions, or allowed to stand in an incubator at 37°C under conditions of 5% CO₂. Note that as for the room temperature conditions, specifically, a plate was placed in a container made of foam polystyrene, and the container was allowed to stand in a room (air conditioning set temperature: 25°C). As for the refrigeration conditions, specifically, the plate was allowed to stand in a refrigerator (set temperature: 4°C).

The state of the aggregate at each temperature condition after 24 hours from the start of standing was observed with an optical microscope. In addition, the number of viable cells of the aggregate was measured using CellTiter-Glo (registered trademark) 3D Cell Viability Assay (Promega Corporation) which measures the ATP amount by a luminescence method. Specifically, the aggregate was collected together with the respective culture medium, and CellTiter-Glo3D Reagent was added in an amount equal to the volume of the culture medium. The admixture was incubated at a room temperature for 30 minutes. After excellent mixing, the luminescence value was measured with a microplate reader (Perkin Elmer).

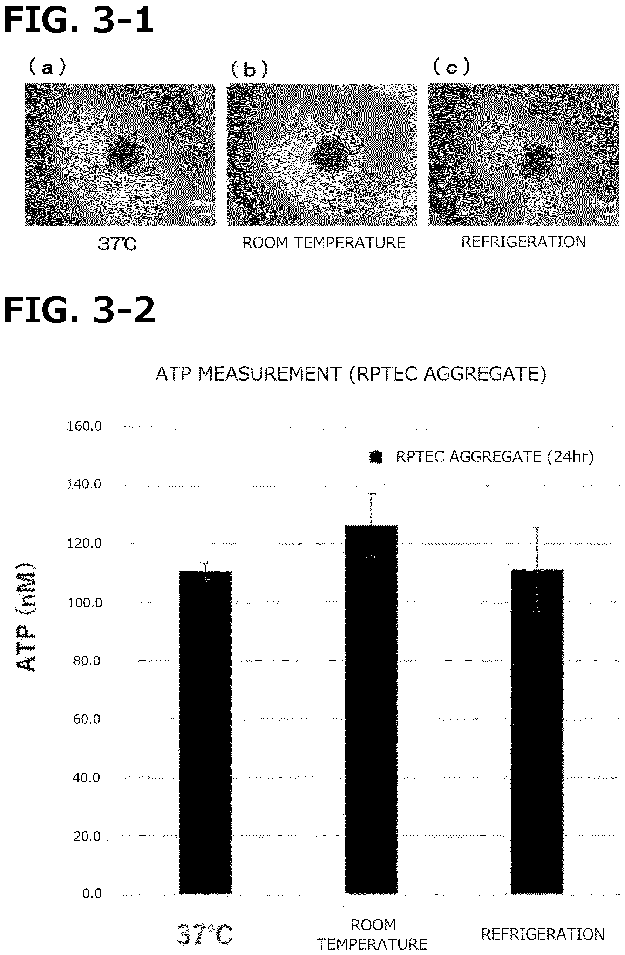

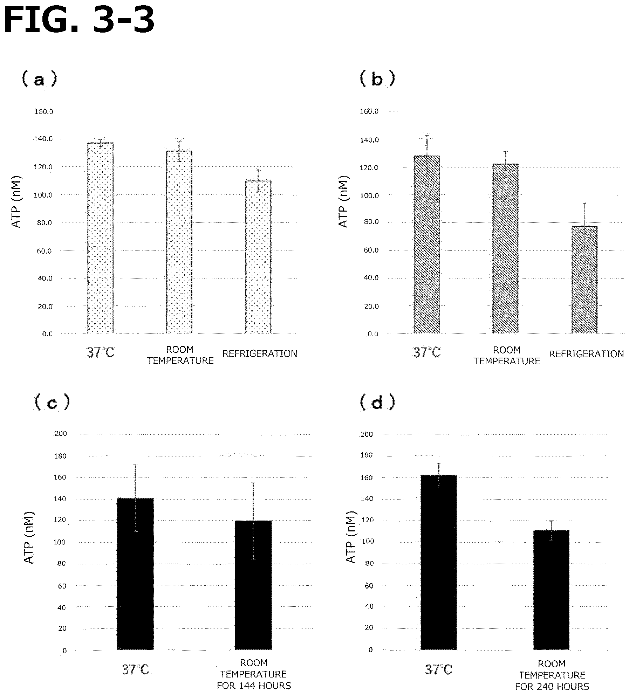

Fig. 3-1 shows the morphology of the aggregate under each temperature condition {(a) 37°C conditions; (b) room temperature conditions; (c) refrigeration conditions}. In addition, Fig. 3-2 shows the results of the ATP amount measurement after standing for 24 hours.

It was confirmed that the aggregate of proximal tubule epithelial cells still maintained the morphology when maintained at a temperature (room temperature or refrigeration) lower than the culture temperature (37°C) for 24 hours. As a result of measuring the ATP amount, the number of viable cells at low temperature was comparable to the number of viable cells at the culture temperature. Since the number of viable cells at low temperature was within the range of the in-facility data of the number of viable cells at the culture temperature, survival maintenance was confirmed.

### <1-2. Time course change of cell viability>

Aggregates were cultured in the same manner as in 1 -1. described above, and a plate containing a culture solution containing the aggregate was allowed to stand under the room temperature conditions or under the refrigeration conditions, or allowed to stand in an incubator at 37°C under conditions of 5% CO₂. After standing, the culture medium was replaced at a frequency of once every two days.

After a lapse of a predetermined time from the start of standing, the ATP amount of the aggregate under each temperature condition was measured in the same manner as in 1-1. described above.

Fig. 3-3 {(a) 48 hours; (b) 72 hours; (c) 144 hours (6 days); (d) 240 hours (10 days)} shows the results of the number of viable cells after lapses of 48 hours, 72 hours, 144 hours, and 240 hours of standing.

It was confirmed that there was almost no difference in the number of viable cells of the aggregate of the proximal tubule epithelial cells after lapses of 48 hours and 72 hours of standing under the room temperature conditions and the number of viable cells under the culture temperature conditions. On the other hand, a decrease in the number of viable cells was confirmed after the lapse of 144 hours of standing under the room temperature conditions and after the lapse of 48 hours of standing under the refrigeration conditions. Table 1 shows the cell viability (ratio of the number of viable cells at each temperature condition to the number of viable cells at the culture temperature) at this time. In Table 1, the cell viability is represented by "A" = 90% or more, "B" = 80% or more and less than 90%, "C" = 50% or more and less than 80%, and "D" = less than 50%. The numerical value in parentheses represents a difference from 100% of each cell viability.

**[Table 1]**

| | 24 Hours | 48 Hours | 72 Hours | 144 Hours (6 Days) | 240 Hours (10 Days) |
|---|---|---|---|---|---|
| Room Temperature | **A** (+14.2%) | **A** (-4.3%) | **A** (-4.7%) | **B** (-15.0%) | **C** (-31.8%) |
| Refrigeration | **A** (+0.6%) | **B** (-19.7%) | **C** (-39.7%) | No data | No data |

From the above results, it was confirmed that the aggregate of renal cells can be maintained at a temperature lower than the culture temperature for 24 hours or longer. In addition, it was confirmed that the aggregate can be stably maintained over a long period of time at a temperature (room temperature) having a small difference from the culture temperature as compared with a lower temperature (refrigeration).

### 2. Analysis of gene expression level of renal cells

### <2-1. Gene expression level in room temperature storage>

The gene expression of the proximal tubule epithelial cell aggregate after the room temperature standing and at the culture temperature was examined and compared with the gene expression in human renal cortex.

Aggregates were cultured in the same manner as in 1-1. described above, and a plate containing a culture solution containing the aggregate was allowed to stand under the room temperature conditions, or allowed to stand in an incubator at 37°C under conditions of 5% CO₂. After standing, the culture medium was replaced at a frequency of once every two days.

mRNA was extracted and purified from each aggregate after the lapse of 72 hours from the start of standing using RNeasy (registered trademark) Mini Kit (QIAGEN N.V). Furthermore, cDNA was synthesized from this mRNA using QuantiTect (registered trademark) Whole Transcriptome Kit (QIAGEN N.V). Using these cDNAs as templates, the gene expression levels of OAT1 (organic anion transporter 1), OCT2 (organic cation transporter 2), and URAT1 (uric acid transporter 1) abundantly expressed in the proximal tubule were measured by a real-time PCR method using Thermal Cycler Dice (registered trademark) Real Time System 1 (Takara Bio Inc.). For all experiments, each sample was measured with n = 3 in one experiment.

For comparison, RNA was extracted in the same manner as described above using human renal cortex collected from a human patient donor, and the gene expression level of OAT1, OCT2, or URAT 1 was measured.

Fig. 4-1 shows the results of the gene expression level of OAT1, Fig. 4-2 shows the results of the gene expression level of OCT2, and Fig. 4-3 shows the results of the gene expression level of URAT1. Table 2-1 shows the comparison of the expression with the human renal cortex and with the culture temperature conditions under the room temperature conditions.

**[Table 2-1]**

| | Room Temperature for 72 Hours (Ratio of GAPDH) | Comparison with 37°C Culture Temperature | Comparison with Human Renal Cortex |
|---|---|---|---|
| OAT1 | 8.3 × 10⁻³ | 86.7% | 40.5% |
| OCT2 | 1.4 × 10⁻² | 53.2% | 121.6% |
| URAT1 | 6.3 × 10⁻⁵ | 217.4% | 24.5% |

In the aggregate of proximal tubule epithelial cells at the culture temperature, the gene expression levels of OAT1, OCT2, and URAT1 were equivalent to those in the human renal cortex. In addition, it was confirmed that in the room temperature condition, there was no change in the gene expression level as compared with the culture temperature conditions and the human renal cortex, and the physiological function of the kidney was still maintained when left standing at the room temperature for 72 hours.

### <2-2. Gene expression level in refrigeration storage>

The gene expression of the proximal tubule epithelial cell aggregate after the refrigeration standing and at the culture temperature was examined and compared with gene expression in human renal cortex.

Aggregates were cultured in the same manner as in 1-1. described above, and a plate containing a culture solution containing the aggregate was allowed to stand under the refrigeration conditions, or allowed to stand in an incubator at 37°C under conditions of 5% CO₂. After standing, the culture medium was replaced at a frequency of once every two days.

From each aggregate after the lapse of 24 hours from the start of standing and the human renal cortex collected from a human patient donor, the gene expression levels of OAT1, OCT2, or URAT1 were measured in the same manner as in 2-1. described above.

Fig. 4-4 shows the results of the gene expression level of OAT1, Fig. 4-5 shows the results of the gene expression level of OCT2, and Fig. 4-6 shows the results of the gene expression level of URAT1. Table 2-2 shows the comparison of the expression with the culture temperature conditions and with the human renal cortex under the refrigeration conditions.

**[Table 2-2]**

| | Refrigeration for 24 Hours (Ratio of GAPDH) | Comparison with 37°C Culture Temperature | Comparison with Human Renal Cortex |
|---|---|---|---|
| OAT1 | 5.3 × 10⁻³ | 120.9% | 25.6% |
| OCT2 | **1.2** × 10⁻² | 107.4% | 104.0% |
| URAT1 | 1.0 × 10⁻⁵ | 88.4% | 3.7% |

From the above results, it was confirmed that the physiological function of the kidney was still maintained when the kidney was left to stand for 24 hours under refrigeration.

### 3. Influence of aggregate cell number

### <3-1. Aggregate cell number>

Renal cells were cultured and collected in the same manner as in 1-1. described above, and then seeded and cultured on a 96 well V-bottom plate subjected to cell low adhesion treatment so that the cell number per well was 125 cells, 500 cells, 2000 cells, 10,000 cells, or 40,000 cells, respectively, to form an aggregate. The aggregate was cultured while replacing the culture medium at a frequency of once every two days.

After culturing for 240 hours or longer, the plate containing the culture solution containing the aggregate was allowed to stand under the room temperature conditions or under the refrigeration conditions, or allowed to stand in an incubator at 37°C under conditions of 5% CO₂. In addition, the state of the aggregate after culturing under each temperature condition was observed with an optical microscope.

The ATP amount of the aggregate under each temperature condition after a lapse of a predetermined time from the start of standing was measured.

Fig. 5-1 shows the morphology of the aggregate in each of the cell numbers {(a) 125 cells; (b) 500 cells; (c) 2000 cells; (d) 10,000 cells; (e) 40,000 cells}. Fig. 5-2 shows the results of the time course measurement of the ATP amount in the aggregate in each of the cell numbers {(a) 125 cells; (b) 500 cells; (c) 2000 cells; (d) 10,000 cells; (e) 40,000 cells}.

When the number of viable cells of the aggregate of proximal tubule epithelial cells was 125 cells to 10,000 cells, there was no significant difference between the room temperature conditions or the refrigeration conditions and the culture temperature condition in any maintenance period. From this, it was confirmed that when the cell number of the aggregate was 125 cells to 10,000 cells, survival could still be maintained after the lapse of 72 hours of standing. On the other hand, when the cell number was 40,000 cells, a decrease in the number of viable cells was confirmed under refrigeration conditions for 48 hours after standing.

Table 3-1 shows the cell viability of the aggregate of each of the cell numbers at this time. The cell viability is represented by "A" = 90% or more, "B" = 80% or more and less than 90%, "C" = 50% or more and less than 80%, and "D" = less than 50%. Note that the numerical value in parentheses represents the difference from the cell viability (100%) at 37°C under each condition.

**[Table 3-1]**

| Cell Number (Cells/Spheroids) | Room Temperature | | | Refrigeration | | |
|---|---|---|---|---|---|---|
| | 24 Hours | 48 Hours | 72 Hours | 24 Hours | 48 Hours | 72 Hours |
| 125 | A (+28.1%) | A (+21.5%) | A (+18.6%) | B (-14.2%) | B (-10.1%) | B (-10.9%) |
| 500 | A (+37.6%) | A (+3.4%) | A (-3.6%) | A (+18.1%) | B (-12.2%) | A (+1.1%) |
| 2000 | A (+47.3%) | A (+26.1%) | B (-11.8%) | A (+12.3%) | A (-0.3%) | A (-7.8%) |
| 10000 | A (+26.7%) | A (+6.7%) | B (-13.5%) | A (+2.3%) | A (-8.0%) | B (-11.7%) |
| 40000 | A (+24.0%) | A (+1.7%) | B (-12.4%) | B (-11.8%) | C (-35.7%) | C (-24.6%) |

### <3-2. Method of calculating maintainable period and maintainable temperature>

From the results of 1-2. and 3-1. described above, it was found that the smaller the difference between the culture temperature and the maintenance temperature, the longer the period until the cell viability decreases. That is, it has been found that there is the following relationship between the maintenance period and the maintenance temperature in the maintenance step. That is, the maintainable period during which the cell viability can be maintained at a predetermined value or more is calculated based on the following formula. (Maintainable period (days)) = A/{(culture temperature (°C)) - (maintenance temperature (°C))}

Here, the value of A is determined according to the desired cell viability and the cell number of the aggregate. For example, according to Table 1, when the cell number of the aggregate is 1000 cells and the culture temperature is 37°C, since the maintainable period during which the cell viability is 90% or more at the maintenance temperature of 25°C is 72 hours (that is, 3 days), A'=36, and since the maintainable period during which the cell viability is 90% or more at the maintenance temperature of 4°C is 24 hours (that is, 1 day), A"=33. The average value 34.5 is defined as A.

Also, for example, according to Table 1, when the cell number of the aggregate is 1000 cells and the culture temperature is 37°C, since the maintainable period during which the cell viability is 80% or more at the maintenance temperature of 25°C is 144 hours (that is, 6 days), A'=72, and since the maintainable period during which the cell viability is 80% or more at the maintenance temperature of 4°C is 48 hours (that is, 2 days), A"=66. The average value 69 is defined as A.

Similarly, the maintainable temperature at which the cell viability can be maintained at a predetermined value or more is calculated based on the following formula. (Maintainable period (days)) = A/{(culture temperature (°C)) - (maintainable temperature (°C))}

Here, A: a coefficient determined according to the predetermined value and the cell number of the aggregate (day ·°C)

From the above formula, the lower limit value of the maintenance temperature according to the desired maintenance period and cell viability can be calculated.

### <3-3. Measurement of diameter and volume of aggregate>

As in 3-1. described above, renal cells were seeded and cultured so that the cell number per well was a predetermined cell number, thereby forming and culturing an aggregate.

After culturing for 10 days or more, the state of each aggregate was observed with a phase contrast microscope BZ-X 710 (Keyence Corporation) and imaged. At this time, all aggregates are submerged in the culture solution. That is, it was confirmed that the specific gravity of each aggregate with respect to the culture medium was 1 or more. The diameter was measured from the morphology photograph using analysis software (Keyence Corporation). Further, the volume was calculated from the obtained diameter, and the volume ratio of the aggregate to the culture medium was calculated. Forty-eight aggregates of each of the aggregates with 10,000 cells and 40,000 cells were collected and weighed, and their specific gravity with respect to the culture medium was calculated.

Table 3-2 shows, in each of the cell numbers, the diameter and volume of the proximal tubule epithelial cell aggregate, and the volume ratio and specific gravity with respect to the culture medium.

**[Table 3-2]**

| Cell Number (Cells/Spheroids) | Diameter (µm) | Volume (mm³) | Volume Ratio to Culture Medium Volume (%) | Specific Gravity |
|---|---|---|---|---|
| 125 | 181.9 | 0.0031 | 0.0026 | ≥ 1.00 |
| 500 | 270.6 | 0.0104 | 0.0086 | ≥ 1.00 |
| 2000 | 428.9 | 0.0413 | 0.0344 | ≥ 1.00 |
| 10000 | 732.8 | 0.2059 | 0.1716 | 1.07 |
| 40000 | 1184.4 | 0.8695 | 0.7246 | 1.11 |

### 4. Effects of temperature on planar cultured proximal tubule epithelial cells

### <4-1. Morphology of planar cultured cells>

Renal cells were cultured and collected in the same manner as in 1-1. described above, and then seeded on a 96 well flat bottom cell culture plate so that the cell number per well was 2 × 10⁴ cells. After seeding, the cells were cultured until they became confluent.

After the culturing, the plate containing the culture solution containing the planar cultured cells was allowed to stand under the room temperature conditions or under the refrigeration conditions, or allowed to stand in an incubator at 37°C under conditions of 5% CO₂. After a lapse of 24 hours of standing, the state of the planar cultured cells under each temperature condition was observed with an optical microscope.

Fig. 6-1 shows the morphology of planar cultured cells under each temperature condition {(a) 37°C conditions; (b) room temperature conditions; (c) refrigeration conditions}. It was confirmed that, in the planar cultured proximal tubule epithelial cells, under the room temperature conditions and the refrigeration conditions, gaps between cells increased, and the morphology of the cells was changed. It was confirmed that, in a case of planar culture, the cell morphology of the proximal tubule epithelial cells was changed by a decrease in culture temperature.

### <4-2. Time course change of cell viability>

Cells were cultured until they became confluent in the same manner as in 4-1. described above. After the culturing, the plate containing the culture solution containing the planar cultured cells was allowed to stand under the room temperature conditions or under the refrigeration conditions, or allowed to stand in an incubator at 37°C under conditions of 5% CO₂. After standing, the culture medium was replaced at a frequency of once every two days.

The ATP amount of the aggregate under each temperature condition after a lapse of a predetermined time from the start of standing was measured. Fig. 6-2 shows the results of the time course measurement of the ATP amount in the aggregate under each temperature condition at each lapse time {(a) 24 hours; (b) 48 hours; (c) 72 hours}.

It was confirmed that the number of viable cells of the planar cultured proximal tubule epithelial cells remarkably decreased at the time point after the lapse of 24 hours of standing under the room temperature conditions and the refrigeration conditions, as compared with the number of viable cells under the culture temperature conditions. Table 4 shows the cell viability (ratio of the number of viable cells under each condition to the number of viable cells at the culture temperature) at this time. The cell viability is represented by "A" = 90% or more, "B" = 80% or more and less than 90%, "C" = 50% or more and less than 80%, and "D" = less than 50%. Note that the numerical value in parentheses represents the difference from the cell viability (100%) at 37°C under each condition.

**[Table 4]**

| | 24 Hours | 48 Hours | 72 Hours | 144 Hours (6 Days) | 240 Hours (10 Days) |
|---|---|---|---|---|---|
| Room Temperature | C (-45.8%) | D (-66.7%) | D (-77.8%) | No data | No data |
| Refrigeration | D (-55.0%) | D (-70.3%) | D (-77.6%) | No data | No data |

### 5. Effects of temperature on aggregate of human iPS cells

Human iPS cells (201B7 strain) were used. The frozen vial of human iPS cells stored in a liquid nitrogen storage was taken out, and the frozen vial was thawed by being immersed in a thermostatic bath at 37°C. After thawing, the cell suspension in the frozen vial was admixed with the recommended culture medium {StemFit (registered trademark), Ajinomoto Co., Inc.} and cultured in a culture dish. The cells were cultured under conditions of 37°C and 5% CO₂ while replacing the culture medium at a frequency of once every day. The cells were collected before becoming confluent, and seeded on a 96 well V-bottom plate {PrimeSurface (registered trademark) plate 96 V, Sumitomo Bakelite Co., Ltd.} subjected to cell low adhesion treatment so that the cell number was 1000 cells per well, and cultured to form an aggregate. The aggregate was cultured while replacing the culture medium at a frequency of once every two days.

After culturing for 240 hours or longer, the plate containing the culture solution containing the aggregate was allowed to stand under the room temperature conditions or under the refrigeration conditions, or allowed to stand in an incubator at 37°C under conditions of 5% CO₂. After a lapse of 24 hours of standing, the state of the cells under each temperature condition was observed with an optical microscope.

The ATP amount of the aggregate under each temperature condition after a lapse of a predetermined time from the start of standing was measured.

Fig. 7-1 shows the morphology of the human iPS cell aggregate under each temperature condition {(a) 37°C conditions; (b) room temperature conditions; (c) refrigeration conditions}. Fig. 7-2 shows the results of the time course measurement of the ATP amount in the human iPS cell aggregate under each temperature condition at each lapse time {(a) 24 hours; (b) 48 hours; (c) 72 hours}.

It was confirmed that the number of viable cells of the human iPS cell aggregate remarkably decreased at the time point after the lapse of 24 hours of standing under the room temperature conditions and the refrigeration conditions, as compared with the number of viable cells under the culture temperature conditions. Table 5 shows the cell viability (ratio of the number of viable cells under each condition to the number of viable cells at the culture temperature) at this time. The cell viability is represented by "A" = 90% or more, "B" = 80% or more and less than 90%, "C" = 50% or more and less than 80%, and "D" = less than 50%. Note that the numerical value in parentheses represents the difference from the cell viability (100%) at 37°C under each condition.

**[Table 5]**

| | 24 Hours | 48 Hours | 72 Hours |
|---|---|---|---|
| Room 1 Temperature | C (-24.1%) | D (-90.1%) | D (-94.3%) |
| Refrigeration | D (-94.8%) | D (-95.6%) | D (-95.8%) |

### (Embodiments of invention)

A first embodiment of the present invention is a method for storing or transporting renal cells, the method including a maintenance step of managing a liquid containing an aggregate of the renal cells to a temperature that is a maintenance temperature and lower than a culture temperature of the renal cells and at which the liquid is not frozen. As a result, there is an effect that renal cells having a high cell viability and maintaining a physiological function of a kidney can be stored or transported at a temperature lower than the culture temperature.

A second embodiment of the present invention is the method according to the first embodiment, in which the maintenance temperature is 0°C or more and less than 37°C. This enhances the effect that cells exhibit high cell viability.

A third embodiment of the present invention is the method according to the first or second embodiment, in which the cell number of one aggregate is 125 cells or more and 10,000 cells or less. This enhances the effect that the aggregate size becomes uniform and furthermore, the cells exhibit high cell viability.

A fourth embodiment of the present invention is the method according to the first to third embodiments, in which a diameter of the aggregate is 100 µm or more and 800 µm or less. This enhances the effect that the aggregate size becomes uniform and furthermore, the cells exhibit high cell viability.

A fifth embodiment of the present invention is the method according to the first to fourth embodiments, in which a volume of the aggregate is 0.001 mm³ or more and 0.300 mm³ or less. This enhances the effect that the aggregate size becomes uniform and furthermore, the cells exhibit high cell viability.

A sixth embodiment of the present invention is the method according to the first to fifth embodiments, in which the number of the aggregate in the liquid is one. As a result, there is an effect that a risk can be reduced that aggregates are joined to each other and a size becomes non-uniform.

A seventh embodiment of the present invention is the method according to the first to sixth embodiments, in which a volume ratio of the aggregate to the liquid is 0.001% or more and 0.200% or less. As a result, there is an effect that the aggregate can be prevented from being exposed to the air due to drying of the liquid during storage or transportation, and oxygen can be sufficiently supplied to the aggregate.

An eighth embodiment of the present invention is the method according to the first to seventh embodiments, in which the specific gravity of the aggregate to the liquid is 1.00 or more and 1.20 or less. As a result, there is an effect that the aggregate is less likely to be affected by changes in the extracellular environment.

A ninth embodiment of the present invention is the method according to the first to eighth embodiments, in which the renal cells are proximal tubule epithelial cells. As a result, there is an effect that an aggregate can be obtained that can be used for pharmacokinetics in renal cells and drug discovery research on renal disease.

A tenth embodiment of the present invention is the method according to the first to ninth embodiments, in which a maintenance period of the maintenance step is equal to or less than a maintainable period in which a cell viability, which is a ratio of the number of viable cells at the maintenance temperature to the number of viable cells at the culture temperature of the renal cells, can be maintained at a predetermined value or more, and the maintainable period is calculated based on a following calculation formula. (Maintainable period (days)) = A/{(culture temperature (°C)) - (maintenance temperature (°C))}

Here, A: a coefficient determined according to the predetermined value and the cell number of the aggregate (day ·°C)

As a result, there is an effect that an upper limit value of the maintenance period can be calculated according to the desired maintenance temperature and cell viability.

An eleventh embodiment of the present invention is the method according to the first to ninth embodiments, in which the maintenance temperature is higher than or equal to a maintainable temperature at which a cell viability, which is a ratio of the number of viable cells at the maintenance temperature to the number of viable cells at the culture temperature of the renal cells, can be maintained at a predetermined value or more, and the maintainable temperature is calculated based on a following calculation formula. (Maintainable period (days)) = A/{(culture temperature (°C)) - (maintainable temperature (°C))}

Here, A: a coefficient determined according to the predetermined value and the cell number of the aggregate (day ·°C)

As a result, there is an effect that a lower limit value of the maintenance temperature can be calculated according to a desired maintenance period and cell viability.

A twelfth embodiment of the present invention is the method according to the tenth or eleventh embodiment, in which when the predetermined value is 90% and the cell number of the aggregate is 1000 cells, the value of A is 34.5. Accordingly, in a case where the cell number of the aggregate is 1000 cells, there is an effect that the maintainable period at the desired maintenance temperature and the maintainable temperature during the desired maintenance period, at which the cell viability is 90% or more, can be calculated.

A thirteenth embodiment of the present invention is the method according to the tenth or eleventh embodiment, in which when the predetermined value is 80% and the cell number of the aggregate is 1000 cells, the value of A is 69. Accordingly, in a case where the cell number of the aggregate is 1000 cells, there is an effect that the maintainable period at the desired maintenance temperature and the maintainable temperature during the desired maintenance period, at which the cell viability is 80% or more, can be calculated.

A fourteenth embodiment of the present invention is the renal cells obtained according to the first to the thirteenth embodiments. As a result, there is an effect that renal cells can be obtained that have a high cell viability and maintain the physiological function of the kidney.

A fifteenth embodiment of the present invention is a drug evaluation system including the renal cells obtained according to the fourteenth embodiment. As a result, there is an effect that a drug evaluation system can be obtained that can be used for pharmacokinetics in renal cells and drug discovery research on renal disease.

A sixteenth embodiment of the present invention is a cell product containing renal cells obtained according to the fifteenth embodiment. This produces an effect of obtaining a cell product that can be used for pharmacokinetics in renal cells and drug discovery research on renal disease.

### Industrial Applicability

The present invention can be used for storage and transportation of a renal cell aggregate that can be used in a drug evaluation system.

### Reference Signs List

- 10: Renal cell storage system
- 20: Renal cell storage device
- 22: Culture vessel
- 24: Liquid
- 26: Aggregate
- 30: Temperature management device
- 40: Storage information input unit
- 50: Temperature management unit
- 52: Maintenance temperature setting unit
- 54: Temperature control unit
- 60: Maintenance period calculation unit
- 70: Display unit

## Claims

1. A method for storing or transporting renal cells, the method comprising
a maintenance step of managing a liquid containing one or more aggregates of the renal cells to a temperature that is a maintenance temperature and lower than a culture temperature of the renal cells and at which the liquid is not frozen.

2. The method according to claim 1, wherein the maintenance temperature is 0°C or more and less than 37°C.

3. The method according to claim 1 or 2, wherein the cell number of one of the aggregates is 125 cells or more and 10,000 cells or less.

4. The method according to any one of claims 1 to 3, wherein a diameter of one of the aggregates is 100 µm or more and 800 µm or less.

5. The method according to any one of claims 1 to 4, wherein a volume of one of the aggregates is 0.001 mm³ or more and 0.300 mm³ or less.

6. The method according to any one of claims 1 to 5, wherein the number of the aggregates in the liquid is one.

7. The method according to claim 6, wherein a volume ratio of one of the aggregates to the liquid is 0.001% or more and 0.200% or less.

8. The method according to any one of claims 1 to 7, wherein a specific gravity of one of the aggregates to the liquid is 1.00 or more and 1.20 or less.

9. The method according to any one of claims 1 to 8, wherein the renal cells are proximal tubule epithelial cells.

10. The method according to any one of claims 1 to 9, wherein
a maintenance period of the maintenance step is equal to or less than a maintainable period in which a cell viability, which is a ratio of the number of viable cells at the maintenance temperature to the number of viable cells at the culture temperature of the renal cells, can be maintained at a predetermined value or more, and
the maintainable period is calculated based on a following calculation formula: (Maintainable period (days)) = A/{(culture temperature (°C)) - (maintenance temperature (°C))}
here, A: a coefficient determined according to the predetermined value and the cell number of one of the aggregates (day·°C).

11. The method according to any one of claims 1 to 9, wherein
the maintenance temperature is higher than or equal to a maintainable temperature at which a cell viability, which is a ratio of the number of viable cells at the maintenance temperature to the number of viable cells at the culture temperature of the renal cells, can be maintained at a predetermined value or more, and
the maintainable temperature is calculated based on a following calculation formula: (Maintenance period (days)) = A/{(culture temperature (°C)) - (maintainable temperature (°C))}
here, A: a coefficient determined according to the predetermined value and the cell number of one of the aggregates (day·°C).

12. The method according to claim 10 or 11, wherein when the predetermined value is 90% and the cell number of one of the aggregates is 1000 cells, the value of A is 34.5.

13. The method according to claim 10 or 11, wherein when the predetermined value is 80% and the cell number of one of the aggregates is 1000 cells, the value of A is 69.

14. A renal cell obtained by the method according to any one of claims 1 to 13.

15. A drug evaluation system comprising the renal cells according to claim 14.

16. A cell product comprising the renal cell according to claim 14.
